(19) **Europäisches Patentamt**
**European Patent Office**
**Office européen des brevets**

(11) **EP 1 810 618 A1**

(12) **EUROPEAN PATENT APPLICATION**

(43) Date of publication:
**25.07.2007 Bulletin 2007/30**

(51) Int Cl.:
***A61B 7/00*** *(2006.01)*

(21) Application number: **06425033.5**

(22) Date of filing: **24.01.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Enel Produzione S.p.A.**
**06100 Roma (IT)**

(72) Inventors:
• **De Michele, Gennaro**
**56100 Pisa (IT)**

• **Guidi, Luca**
**57125 Livorno (IT)**
• **Sello, Stefano**
**56123 Pisa (IT)**
• **Nicolino, Ambrosino**
**57125 Livorno (IT)**
• **Strambi, Soo-Kyung**
**56100 Pisa (IT)**

(74) Representative: **Bardini, Marco Luigi et al**
**Società Italiana Brevetti SpA**
**Corso dei Tintori 25**
**I-50122 Firenze (IT)**

(54) **System and method of processing acoustic signals for the energy characterisation of the respiratory condition**

(57) A method of processing acoustic signals for the characterisation and diagnosis of the respiratory condition in a patient, wherein an energy analysis is performed on these signals based on the wavelet transform, suitable for calculating energy values and their frequency distribution, said values being indicative of the respiratory condition in said patient. A statistical analysis is then performed on the average wavelet spectra for the purpose of gaining precise information on the function of energy distribution of the respiratory signal which is considered a clear indication of the respiratory condition of a given subject. In this way it is possible to follow the evolution in time of the degree of the illness or the efficacy of a treatment on the basis of the variation in energy parameters.

**EP 1 810 618 A1**

**Description**

The present invention relates to a system and to a method of processing acoustic signals (called E.L.S.A.) from the respiratory system, useful in the diagnosis of the degree of gravity of respiratory pathologies and in particular suitable for providing an objective characterisation of the optimal state of mechanical ventilation which a given subject has to undergo.

The methods currently used to assess the degree of gravity of a respiratory pathology are basically of the clinical and/or instrumental type.

The first type includes evaluations of dyspnoea and exercise capacity.

The instrumental evaluations detect the values of haematic gases, static and dynamic lung volumes, the diffusion capacity of the alveolar-capillary membrane, the respiratory muscle function, the pressure/volume ratios of the thoracic-pulmonary system and the ratios between ventilation and perfusion.

Moreover, like the majority of biomedical signals, respiratory signals are also of the non-stationary type with highly complex time-frequency characteristics. The most commonly used mathematical method for the analysis of signals in biomedical applications is the Fourier transform which breaks down a given signal into its frequency components.

However this technique requires the signal at issue to be of the stationary type, i.e. without variations in time of its frequency content, whereas, as mentioned, biomedical signals are generally non-stationary, with an intermittent and variable frequency content.

The limits of the Fourier analysis can be partly overcome using a mobile-window Fourier transform, also known as Gabor transform, which assumes that the signal be almost stationary in a short time interval. However this method too entails limitations and inaccuracies, in particular during the signal partition steps, so that the Gabor transform is disadvantageous for the analysis of signals involving different frequency ranges.

The object of the present invention is to solve the aforesaid problems of the known art by providing a method of processing signals from the respiratory system as claimed in claim 1.

The present invention also relates to a system as claimed in the independent claim 11, suitable for implementation of the aforesaid method of processing.

An additional object of the present invention is a computer product, that is to say a software program specially studied for implementation in an integrated system of acquisition of respiratory signals.

According to one aspect of the present invention a method is provided that is suitable for calculating parameters, both of the quality and quantity type, that are particularly useful in the assessment of the level of efficiency of the respiratory system.

This is carried out through a special analysis of the acoustic signals: an energy analysis based on the wavelet transform.

The main advantage of the method according to the present invention is that, through the wavelet energy analysis performed on a series of acoustic signals acquired in succession, it is possible to obtain information on the average energy content and its distribution at the various characteristic frequencies. This item of information already allows a quality evaluation of the state of efficiency of the respiratory system. For a quantity evaluation the method according to the invention involves the extraction of numerical indicators (such as the quartiles or total energy at different frequency bands) that are univocally correlated to the condition of the subject in question and hence to the possible level of gravity of the pathology.

An important advantage comes from the fact that processing of the wavelet maps and the calculation of the energy information can be performed within a dedicated hardware system, advantageously connected to the signals acquisition equipment, enabling the obtaining, practically in real time, of information on the condition of the patient and fast evaluation of the effect of pharmacology or ventilation treatments.

Further advantages, features and use of the present invention will be made clearer by the following detailed description of one of its preferred embodiments, given by way of a non-limiting example and referring to the figures of the accompanying drawings, in which:

Figure 1 is a block diagram of a system for the energy characterisation of the respiratory condition according to the present invention;
Figure 2 is a flow diagram of the method of processing of acoustic signals from the respiratory system (E.L.S.A.) according to the present invention;
Figure 3 illustrates use of the equipment used in the method according to the invention;
Figures 4 and 5 show the results obtained by applying the processing method according to the invention to a single signal from a healthy subject and to a patient respectively;
Figures 6 and 7 show the results obtained by applying the processing method according to the invention to a sequence of signals from a healthy subject and a patient respectively;
Figure 8 shows the results of the statistical analysis for each subject;

Figure 9 illustrates a possible graphic form of use of the statistical parameters obtained with the method according to the invention to distinguish pathological conditions from normal conditions.

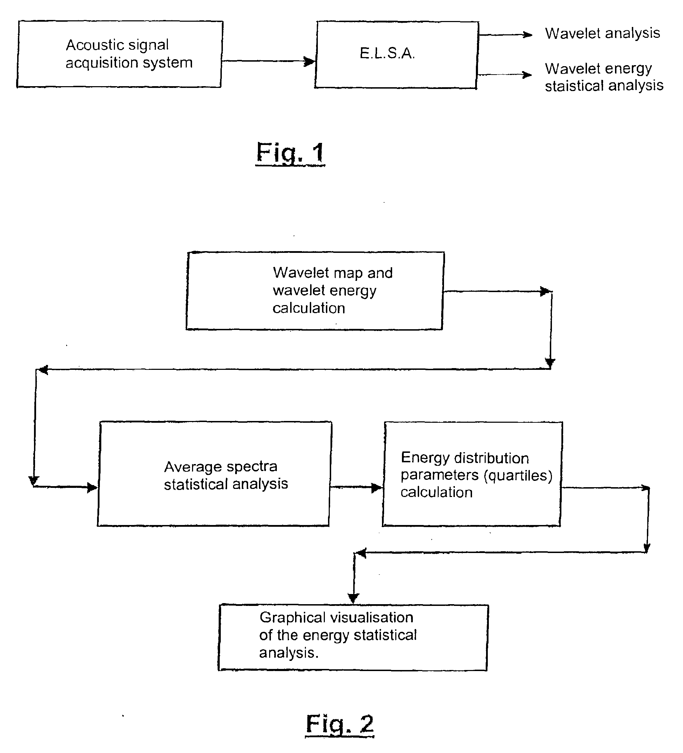

Referring to Figure 1, a system according to the present invention comprises means for the acquisition of acoustic signals for the respiratory system. A central control unit comprising means for the processing of data receives these signals in input, processes them and supplies in output corresponding information relating to predetermined parameters in a format suitable for being displayed on a monitor, printed or reproduced in any other way.

According to a first embodiment of the system according to the present invention the analysis and processing of the input signals (identifying the acoustic signals) is performed off line.

Dedicated equipment is used for the acquisition of acoustic signals, developed in a such a way as to acquire the acoustic signal in an optimal manner, and able to store the signals acquired in a digital format in the form of files on any kind of support (floppy disk, CD or other). More specifically the equipment comprises a recording system with a contact sensor with cavity (microphone positioned inside an acoustical bell) designed specifically to detect acoustic signals with a good signal/noise ratio, a two-channel amplifier with gain that can be selected from 1-2-5-10-20-50-100, band-pass filter with a cut frequency lower than 100 Hz and higher than 3000 Hz and an external audio card used to avoid problems of electronic noise. The signals are then acquired via a PC wherein a specific software program has been developed in Labview language for a first treatment and filing of the signals.

The files are subsequently read by the control unit which launches the processing procedures in order to obtain maps and state indicators as mentioned previously.

All the processing steps of the method according to the present invention are implemented via software through specific procedures provided thereby.

The method is to be dealt with completely from an analytical standpoint herein below in the description, while a detailed description will not be given of any special software implementation of the same method, the latter possibly depending on the hardware platform used, the programming language chosen and also different approaches in the numerical implementation of the algorithms, all parameters within the reach of a person skilled in the art.

In a second preferred embodiment a system in accordance with the present invention instead comprises the instrumentation for the acquisition of the acoustic signals, and their transfer in real time to the central unit which processes them in real time and instantaneously supplies the results of the analysis.

The subsequent Figure 2 is a flow diagram of the processing method according to the present invention.

The macro operations performed on the input data are represented in sequence in the diagrams in Figure 2.

At a first step S1 the wavelet transform of the acoustic signals is calculated.

The wavelet analysis is preferably applied iteratively on a sample of acoustic signals, acquired during the session of measurement of the same signals. In general the analysis of 10 signals is sufficient for an adequate statistical evaluation (step S2) of the results that shows the most probable values and the respective uncertainty of the integral wavelet quantities calculated.

These statistical analyses lead to the determination of energy parameters (average wavelet spectra) and various correlated global indicators.

This allows the display (step S4) of the morphology of the most representative energy content for a given subject as a function of the frequency and the performing of quality comparisons between different subjects or for a given subject over time.

This allows, in particular, an evaluation with high sensitivity of the effect of a given evaluation or therapeutic treatment (pharmacological, surgical or ventilatory) in subjects with various respiratory pathologies using the acoustic signals directly.

In the following a detailed mathematical treatment is given to describe the calculation algorithm that has been used.

As is known, the continuous wavelet transform represents an optimal localized decomposition of a series, *x(t)*, as a function of both time *t* and a scale variable (frequency) $\alpha$:

$$W_f(a, \tau) = \frac{1}{\sqrt{a}} \int_{-\infty}^{+\infty} x(t)\psi^*(\frac{t-\tau}{a})dt,$$

where $\psi$ is a wavelet function verifying the following admissibility condition:

$$C_\psi = \int_{-\infty}^{+\infty} |\hat{\psi}(\omega)|^2 |\psi|^{-1} d\omega < \infty$$

where: $C_\psi$ and $\psi(\omega)$ are the admissibility constant and the Fourier transform of the wavelet function, respectively.

The parameters $\alpha$ and $\tau$ denote the dilation (scale factor) and translation (time parameter), respectively.

The local wavelet spectrum is then defined as:

$$P(k,t) = \frac{1}{2C_\psi k_0} |W(\frac{k_0}{k}, t)|^2, \ k \geq 0$$

where $k_0$ denotes the peak frequency of the analyzed wavelet function $\psi$.

From the local wavelet spectrum a mean or global wavelet spectrum $P(k)$ can be derived:

$$P(k) = \int_{-\infty}^{+\infty} P(k,t) dt$$

which is related to the total energy $E$ of the signal $x(t)$ by:

$$E = \int_{0}^{+\infty} P(k) dk$$

The relationship between the ordinary Fourier spectrum $P_F(\omega)$ and the global wavelet spectrum $P(k)$ is given by:

$$P(k,t) = \frac{1}{C_\psi k} \int_{0}^{+\infty} P_F(\omega) |\hat{\psi}(\frac{k_0 \omega}{k})|^2$$

indicating that the global wavelet spectrum is the average of the Fourier spectra weighted by the square of the Fourier transforms of the analysed wavelet $\psi$ shifted at frequency $k$.

To the end of the present invention the Morlet wavelet function has been advantageously selected which allows to get amplitude and phase information on the signals under examination at a given scale $\alpha$:

$$\psi(t) = e^{ik_0 t} e^{-t^2/2}$$

For the purposes of the present invention the average energy content obtained by the average wavelet spectrum can be considered as the main variable. Through this quantity important information can be obtained on the energy distribution at the various frequencies (and therefore spatial scales too).

In order to validate the method described hitherto, specific tests have been performed both on patients (with primary or secondary pneumological pathologies) and on healthy subjects. A total of 27 patients and 10 healthy subjects were taken into consideration.

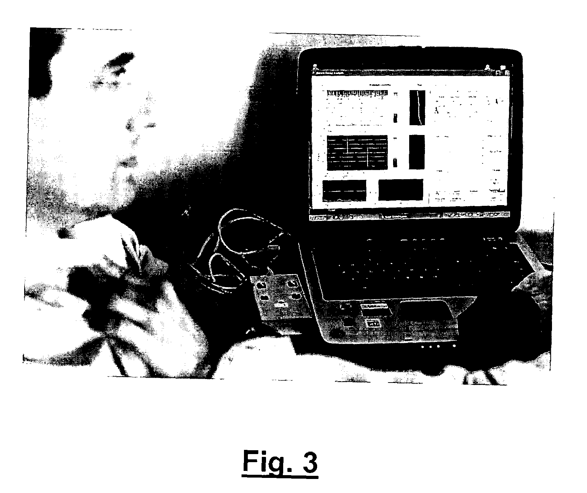

The experiments were performed in a manner to be described herein below, also referring to Figure 3.

4

All the subjects who underwent the test were requested to perform a spirometry test. In the same test session the vital parameters were measured, such as respiratory frequency, cardiac frequency and blood pressure. The dyspnoea was also evaluated by means of the Borg scale and a blood sample taken for analysis of the haematic gases. Finally the respiratory sound was recorded at rest, with non-invasive mechanical ventilation either before and after pharmacological treatment or before and after a surgical operation. The recording consisted in resting a microphone on the suprajugular cavity for about 30 seconds and making the subject concerned breathe spontaneously.

This operation was repeated ten times at regular intervals of 1 minute to extract the statistically significant results correlated to the signals recorded. All the tests in which sound interference or artefacts were noted in the acoustic signal were not stored.

More particularly the clinical protocol followed is set forth in detail herein below.

a) Study of patients with chronic obstruction of the respiratory tract.

Subjects: 40 patients with spirometric obstructive syndrome with significant variation (at least 12% increase or 200 ml) of the $FEV_1$ after administration of a bronchodilator. Patients already subjected to spirometric tests for at least 2 years, able to perform the manoeuvre correctly and repeatably.

Pathology: COBP and asthma at any stage of the illness (stability and/or further worsening).
Setting: hospital ward and/or outpatients.
Protocol:

- Basal spirometry (forced manoeuvre, the best of repeatable three) performed without inhalation treatment (suspension of long-acting $\beta_2$ and inhalatory steroids for at least 12 hours, short-acting for at least 4 hours; slow-release theophylline for at least 12 hours).
- Basal evaluation of the E.L.S.A.
- Administration of the pharmacological treatment of 400 mcg (salbutamol, administrated with a spacer).
- Spirometry (forced manoeuvre, the best of repeatable three) performed 15 minutes after inhalation of salbutamol as inhalation treatment.
- Evaluation of the post-bronchodilatory E.L.S.A.

b) Study of patients with mechanical ventilation.

Subjects: 40 patients with invasive or non-invasive mechanical ventilation.
Pathology: acute or chronic respiratory deficiency.
Setting: respiratory intensive care unit.
Protocol:

- Basal evaluation of the E.L.S.A. during spontaneous breathing.
- Administration of mechanical ventilation.
- Evaluation of the E.L.S.A. during mechanical ventilation.
- Final evaluation of the E.L.S.A. during spontaneous breathing.

The acoustic signals were recorded by means of an integrated system, comprising a system of pre-treatment of the acoustic signals. The sound activity was measured by means of an acoustical bell suitable for acquiring good-quality signals: each signal of each single acquisition had to be repeatable. The recordings of the acoustic signals were amplified and filtered, converted from analogue format to a digital format (sampling at 8000 Hertz, anti-aliasing filtering, with resolution of 8 bits) and stored in the computer in the form of files.

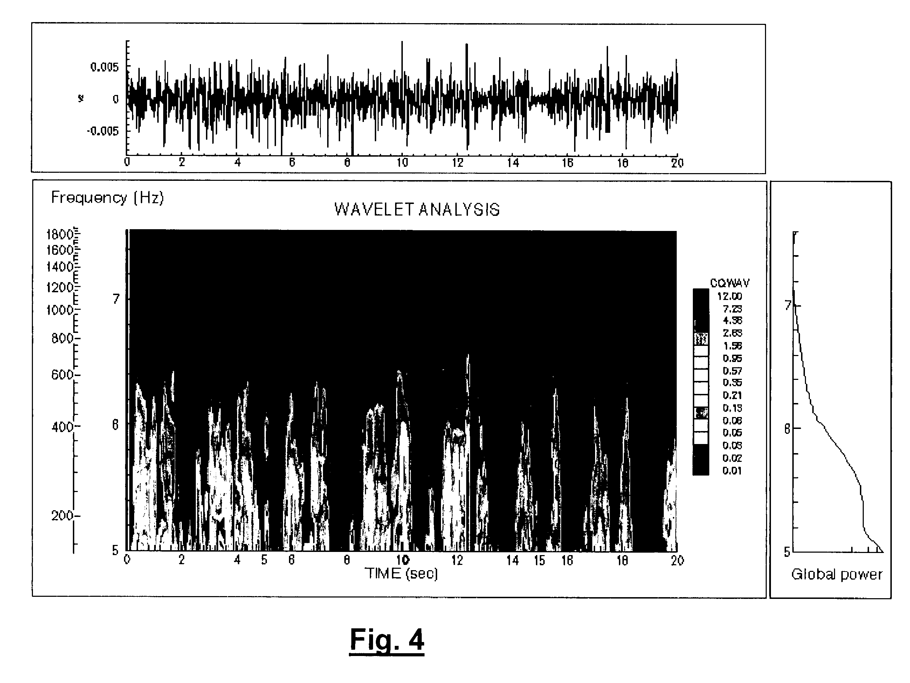

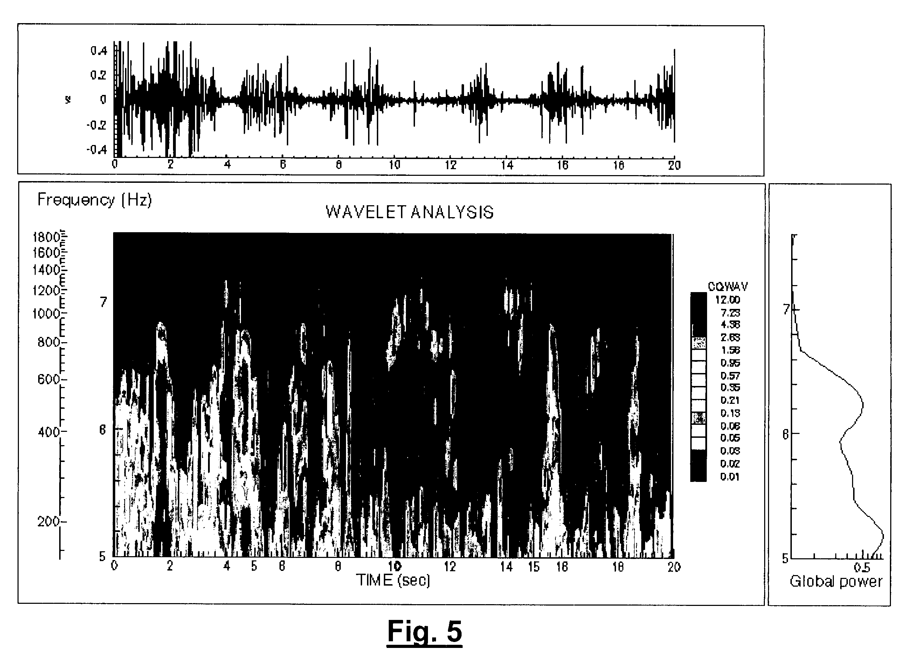

For the purposes of the analysis the acoustic activity taken from the surface at the suprajugular cavity was recorded, as shown in Figure 3. Moreover a diagnosis was given for all patients based on clinical and instrumental tests. The subsequent Figure 4 shows an example of the results obtained by applying the processing method according to the present invention to a single acoustic signal recorded by a healthy subject. Figure 5 shows the same information for a patient.

Both Figures show the wavelet analysis deriving from the acoustic signals.

The central panel displays the wavelet power map depicted using a colour scale for the energy intensity of the signal.

The horizontal axis displays the time variable expressed in seconds and the vertical axis shows the frequency values according to a logarithmic scale.

The right-hand panel shows the global power spectrum, or the energy content, obtained through integration over time of the corresponding local power spectrum. The selected range of frequencies is comprised between 150 and 1800 Hz, as this range contains all the significant components of the acoustic energy of breathing. At first sight, using solely a quality criterion, some important characteristics, typical of the two different classes of cases, can already be obtained: for the healthy subject the energy distribution is more limited in the medium-low frequencies and has a more regular trend.

On the other hand, for the patient the power content shows a more irregular energy distribution and displaced towards the higher frequencies.

The results obtained with the present invention show that the variation of the distribution of the energy content demonstrated through the wavelet analysis is an expression of the variation in the respiratory condition. In order to obtain reliable results a statistical analysis is advantageously performed on all the wavelet spectra. The statistical procedure used is the following:

- the average spectrum is calculated from the power spectra of the individual tests, considering the values of the single curves for each frequency analysed.

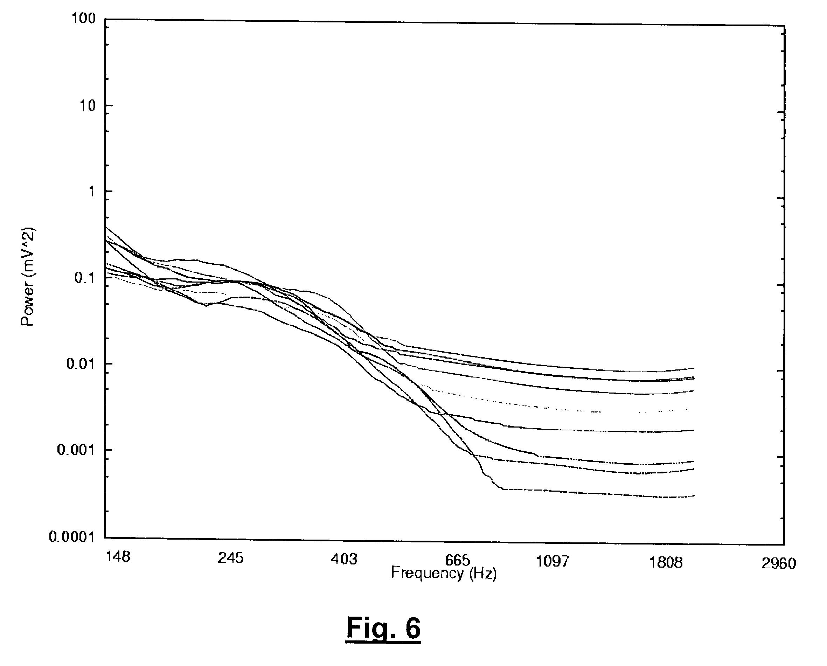

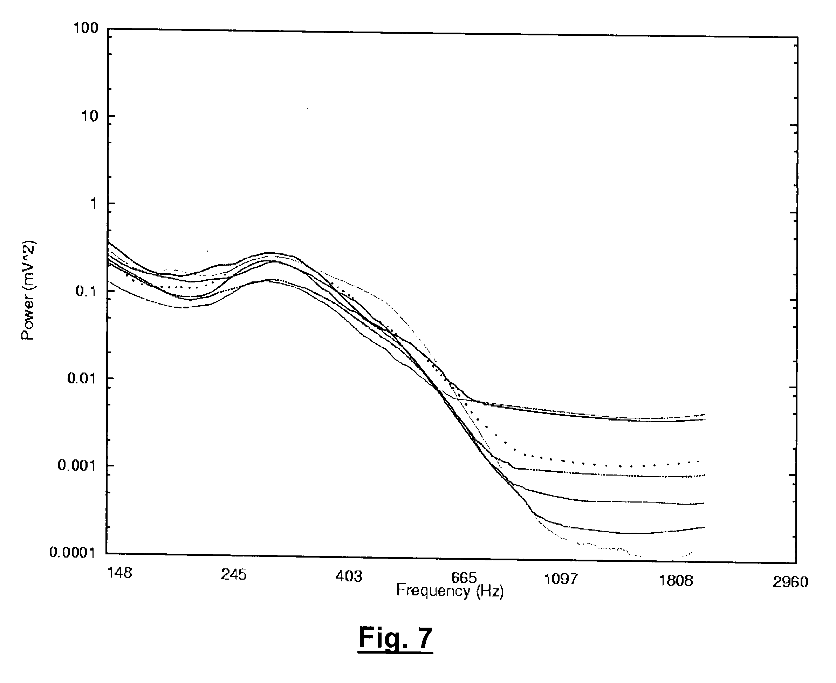

The subsequent Figure 6 shows an example of the results obtained by applying the processing method according to the present invention to a sequence of acoustic signals recorded by a healthy subject. Figure 7 shows the same information for a patient.

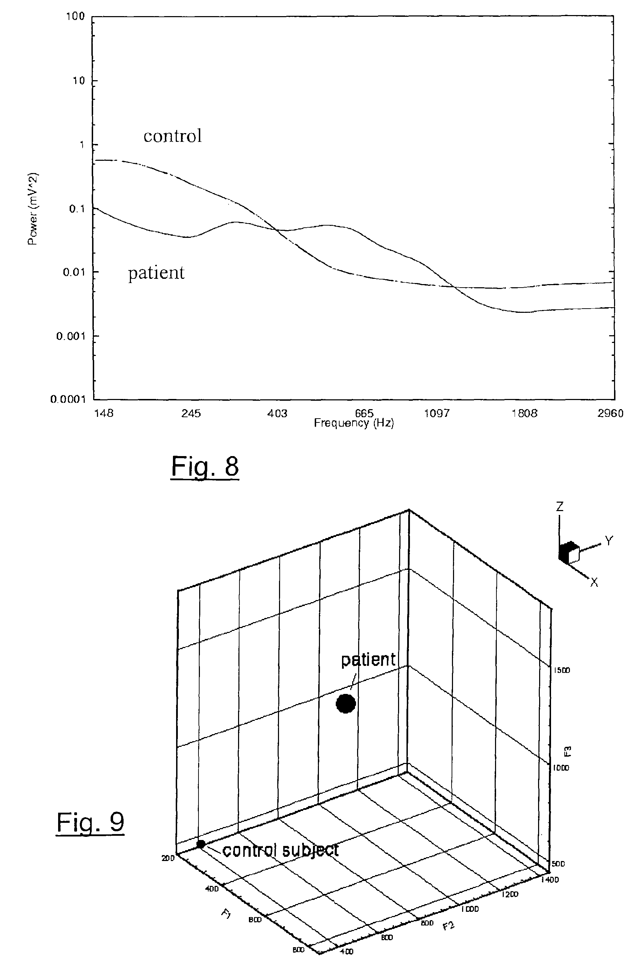

It is thus possible to show in a graph (Figure 8) the results of this statistical analysis through the global wavelet power for each of the subjects examined.

In order to characterise quantitatively the distribution of energy in the various frequency bands analysed, the frequency spectrum is divided into parts in such a way that each of them contains the same energy quantity.

The quartiles $f_{25\%}$, $f_{50\%}$, $f_{75\%}$ which divide the power spectrum into four equal parts are parameters already used in the analyses documented in literature. They supply an evaluation of the balancing of energy between high and low frequencies and can therefore be used to characterise the overall variations of the different respiratory types. Using these parameters in a three-dimensional representation, where each point of the space is defined by the vector whose components are the values of the three aforesaid quartiles, it is possible to distinguish correctly the presence of pathological situations, allowing a clear and direct classification, compared with the previous clinical evaluations (Figure 9).

Through the wavelet technique described above it is therefore possible to follow the evolution in time of the degree of the illness in a given patient, or the efficacy of an instrumental treatment, on the basis of the variations in the energy parameters. In this respect it is particularly useful to perform the forced ventilation check using an "intelligent" system, capable of adapting automatically to the various situations, and varying the energy parameters optimally.

The present invention has been described hitherto according to its preferred embodiments, given by way of a non-limiting example.

It is understood that other forms are foreseen, all to be considered as within the scope of the invention, as set forth in the annexed claims.

## Claims

1. Method of processing of acoustic signals for the characterisation and diagnosis of the respiratory condition in a patient, **characterised in that** it comprises a first processing step, based on a wavelet analysis of said acoustic signals suitable for calculating energy values and their frequency distribution, said values being indicative of the respiratory condition in said patient, and a second step comprising a statistical analysis of the average wavelet spectra in order to gain specific information on the function of energy distribution of the respiratory signal that is considered a clear indication of the respiratory condition of a given subject.

2. Method according to claim 1, comprising a step for providing a signal $f(t)$ relating to the respiratory sound at the tracheal level of said patient.

3. Method according to claims 1 or 2, wherein said wavelet analysis comprises the calculation of a wavelet transform $W_f(a, \tau)$ for each of said acoustic signals $f(t)$.

4. Method according to claim 3, wherein the wavelet transform $W_f(a, \tau)$ is based on the Morlet function.

5. Method according to claim 3 or 4, wherein said analysis comprises the calculation, for each signal, of a continuous wavelwt transform according to the relationship

$$W_f(a, \tau) = \frac{1}{\sqrt{a}} \int_{-\infty}^{+\infty} x(t) \psi^*(\frac{t - \tau}{a}) dt,$$

.

6. Method according to claim 5, wherein said analysis comprises the calculation of a local wavelet spectrum according to the relationship:

$$P(k, t) = \frac{1}{2C_\psi k_0} | W(\frac{k_0}{k}, t) |^2, \ k \geq 0$$

7. Method according to claim 6, wherein said analysis comprises the calculation of a global wavelet spectrum via an integration of the local wavelet spectrum in relation to the variable $\tau$.

8. Method according to claim 7, wherein said analysis comprises the calculation of a global energy value obtained by means of an integration of the global wavelet cross-spectrum in relation to the variable *a*.

9. Method according to any one of the previous claims, comprising a step with application of a first statistical model to the set of acoustic signals $(f(t))_i$ acquired for the same patient, wherein the set of wavelet information for each single signal is analysed on a statistical basis to obtain an average wavelet spectrum representing all the tests performed.

10. Method according to claim 7, comprising a step of application of a second statistical model to the set of global wavelet spectra calculated, comprising the extraction of energy distribution parameters (quartiles) on the average wavelet spectrum to obtain a clear and accurate characterisation of the respiratory condition of the subject in question.

11. System of processing of acoustic signals *f(t)* from the respiratory system of a patient for the characterisation and diagnosis of the respiratory condition of said patient, **characterised in that** it comprises means for the acquisition of said acoustic signals *f(t)* in a digital form, and means of processing said acoustic signals suitable for performing a wavelet analysis thereof, said analysis being suitable for determining global energy values, said values being indicative of the state of efficiency of respiration in said patient.

12. System according to claim 11, wherein said means of acquisition comprise a system for detecting said acoustic signals.

13. System according to claim 11 or 12, also comprising analogue-digital conversion means and pre-treatment of said signals acquired.

14. System according to any one of claims 11 to 13, wherein said analogue-digital conversion and pre-treatment take place in real time.

15. System according to any one of claims 11 to 14, wherein said converted and pre-treated signals are acquired in real time by said acquisition means.

16. System according to any one of claims 11 to 15, wherein said acquisition means acquire at least one acoustic signal *f(t)*, each signal relating to the sound detected at the trachea level of said patient.

17. System according to any one of claims 11 to 16, wherein said processing means are suitable for performing the calculation of a wavelet transform $W_f(a, \tau)$ for each of said acoustic signals *f(t)*.

18. System according to claim 17, wherein each wavelet transform $W_f(a, \tau)$ is based on the Morlet function.

19. System according ro claim 17 or 18, wherein said processing means are capable of carrying out the calculation, for each signal, of a wavelet transform according to the relationship:

$$W_f(a, \tau) = \frac{1}{\sqrt{a}} \int_{-\infty}^{+\infty} x(t) \psi^*(\frac{t - \tau}{a}) dt,$$

**20.** System according to claim 19, wherein said processing means are capable of carrying out the calculation of a local wavelet spectrum according to the relationship:

$$P(k, t) = \frac{1}{2C_\psi k_0} | W(\frac{k_0}{k}, t) |^2, \; k \geq 0$$

**21.** System according to claim 20, wherein said processing means are capable of carrying out the calculation of a global wavelet spectrum by integration of the local wavelet spectrum with respect to the variable $\tau$.

**22.** System according to claim 21, wherein said processing means are suitable for performing the calculation of energy parameters obtained via analysis of the global wavelet spectra.

**23.** System according to any one of claims 11 to 22, wherein said processing means are suitable for applying a first statistical model to the set of acoustic signals acquired for the same patient, wherein the set of wavelet information for each individual signal is analysed on a statistical basis to obtain an average wavelet spectrum representing all the tests performed.

**24.** System according to claim 23, wherein said processing means are suitable for applying a second statistical model to the set of wavelet spectra calculated, comprising the extraction of energy distribution parameters (quartiles) on the average wavelet spectrum to obtain a clear and accurate characterisation of the respiratory condition of the subject in question.

**25.** System according to any one of claims 12 to 24, wherein said processing means comprise one or more software modules.

**26.** Computer product, in particular a program, stored on a storage medium or functioning on a data processing system, suitable for implementing a method according to any one of claims 1 to 10.

```
┌─────────────────────┐          ┌─────────────────┐      → Wavelet analysis
│  Acoustic signal    │          │                 │
│  acquisition system │ ───────→ │    E.L.S.A.     │      → Wavelet energy
│                     │          │                 │        staistical analysis
└─────────────────────┘          └─────────────────┘
```

## Fig. 1

```
         ┌──────────────────────┐
         │  Wavelet map and     │
         │  wavelet energy      │ ──────────────────┐
         │  calculation         │                   │
         └──────────────────────┘                   │
     ┌───────────────────────────────────────────────┘
     │
     │   ┌──────────────────────┐   ┌──────────────────────────┐
     │   │                      │   │  Energy distribution     │
     └─→ │  Average spectra     │ → │  parameters (quartiles)  │ ──┐
         │  statistical analysis│   │  calculation             │   │
         │                      │   │                          │   │
         └──────────────────────┘   └──────────────────────────┘   │
                            ┌──────────────────────────────────────┘
                            │
              ┌─────────────────────────┐
              │  Graphical visualisation│
              │  of the energy statistical│
              │  analysis.              │
              └─────────────────────────┘
```

## Fig. 2

**Fig. 3**

**Fig. 4**

EP 1 810 618 A1

**Fig. 5**

EP 1 810 618 A1

**Fig. 6**

13

**Fig. 7**

EP 1 810 618 A1

## Fig. 8

## Fig. 9

European Patent
Office

**EUROPEAN SEARCH REPORT**

Application Number

EP 06 42 5033

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MACEY K E ET AL: "Calculating rhythmicity of infant breathing using wavelets" PROCEEDINGS OF THE SPIE - THE INTERNATIONAL SOCIETY FOR OPTICAL ENGINEERING SPIE-INT. SOC. OPT. ENG USA, vol. 4119, 2000, pages 1002-1010, XP002389711 ISSN: 0277-786X | 1,3-13, 17-26 | INV. A61B7/00 |
| Y | * the whole document * | 2,14-16 | |
| Y | NISSAN M ET AL: "A microcomputer based lung sounds analysis." COMPUTER METHODS AND PROGRAMS IN BIOMEDICINE. MAY 1993, vol. 40, no. 1, May 1993 (1993-05), pages 7-13, XP002389712 ISSN: 0169-2607 * the whole document * | 2,14-16 | |
| X | KANDASWAMY A ET AL: "Neural classification of lung sounds using wavelet coefficients." COMPUTERS IN BIOLOGY AND MEDICINE. SEP 2004, vol. 34, no. 6, September 2004 (2004-09), pages 523-537, XP002389713 ISSN: 0010-4825 | 1,3-13, 17-26 | TECHNICAL FIELDS SEARCHED (IPC)  A61B |
| Y | * pages 525-530 * | 2,14-16 | |
| A | J.E. EARIS, B.M.G. CHEETHAM: "Current methods used for computerized respiratory sound analysis" EUROPEAN RESPIRATORY REVIEW, ISSN 0905-9180, vol. 10, no. 77, 2000, pages 586-590, XP002389714 United Kingdom * page 588, signal processing * | 1-25 | |

-/--

The present search report has been drawn up for all claims

| Place of search | Date of completion of the search | Examiner |
|---|---|---|
| The Hague | 11 July 2006 | Lommel, A |

**European Patent Office**

**EUROPEAN SEARCH REPORT**

Application Number

EP 06 42 5033

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PERRIER V ET AL: "Wavelet spectra compared to Fourier spectra" JOURNAL OF MATHEMATICAL PHYSICS USA, vol. 36, no. 3, March 1995 (1995-03), pages 1506-1519, XP002389715 ISSN: 0022-2488 * II. wavelet and fourier spectra, definitions and properties * ----- | 1-25 | |

TECHNICAL FIELDS SEARCHED (IPC)

The present search report has been drawn up for all claims

| Place of search | Date of completion of the search | Examiner |
|---|---|---|
| The Hague | 11 July 2006 | Lommel, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P04C01)